# FASCICULE DE BREVET EUROPEEN

(11) **EP 1 431 739 B1**
(45) Date de publication et mention de la délivrance du brevet: **19.07.2006**
(21) Numéro de dépôt: 03293034.9
(22) Date de dépôt: 04.12.2003
(51) Int. Cl.: G01M 3/38, G01N 21/35, G01V 9/00

(54) **Dispositif optique de détection de gaz dans l'air et système terrestre mobile associé**
Optische Vorrichtung zum Nachweis von Gas in der Luft und zugehöriges erdgebundenes, mobiles System
Optical device for detecting gas in the air and associated terrestrial, mobile system

(30) Priorité: 17.12.2002 FR 0216010
(43) Date de publication de la demande: 23.06.2004
(73) Titulaire: Gaz de France (GDF) Service National, 75017 Paris (FR)
(72) Inventeur: Barejoux, Alain, 93130 Noisy le Sec (FR); Cagnon, Francois, 75018 Paris (FR); Liffraud, Gérard, 95570 Bouffemont (FR); Bournazaud, Frédérique, 75017 Paris (FR)
(74) Mandataire: Thinat, Michel

(56) Documents cités:
- FR-A- 1 382 182
- US-A- 5 946 095
- US-A- 6 116 097
- US-B1- 6 464 392

## Description

L'invention concerne en général l'industrie du gaz, en particulier du gaz naturel.

Plus précisément, l'invention concerne, selon un premier aspect, un système terrestre mobile de détection in situ d'un ou plusieurs gaz dans l'air, tel que du méthane, à proximité du sol, comprenant un véhicule apte à se déplacer sur le sol, et un dispositif optique de détection du gaz rigidement fixé sur le véhicule, ce dispositif de détection comprenant un émetteur fixe produisant un faisceau optique, un récepteur fixe captant le faisceau optique après que celui-ci ait parcouru un chemin prédéterminé, et des moyens pour détecter et quantifier le gaz sur ledit chemin en fonction des caractéristiques du faisceau capté par le récepteur.

Des systèmes de ce type sont connus de l'art antérieur, et notamment des documents de brevet US 5,742,053 et de US 5,946,095.

Chacun d'eux révèle une voiture automobile équipée d'un dispositif de détection infrarouge fixé au niveau du pare-chocs avant du véhicule.

Cette disposition du dispositif de détection présente plusieurs désavantages. L'émetteur et le récepteur étant fragiles, ils doivent être placés suffisamment haut pour ne pas heurter la chaussée ou des obstacles faisant saillie sur celle-ci, comme par exemple des dos d'ânes ou des bordures de trottoirs. Le faisceau se trouve ainsi relativement loin du sol.

Or l'une des applications principales du système consiste à détecter les émanations de gaz venant du sol, qui peuvent être liées à des fuites du réseau souterrain de distribution de gaz naturel. Le gaz est fortement concentré au ras du sol, mais sa concentration décroît rapidement quand on s'éloigne du sol. La position du dispositif de détection de US 5,742,053 et de US 5,946,095 est défavorable dans ce contexte.

Par ailleurs, des dispositifs de détection dans cette position peuvent être dangereux pour les piétons en cas de choc, et l'utilisation d'un véhicule ainsi équipé est soumise à autorisation par la législation en Europe.

Par ailleurs, le document US-A-6 116 097 décrit des moyens permettant de détecter des produits chimiques se trouvant sur un sol. Ces moyens comprennent, montés sur un véhicule, au moins un garde-boue disposé derrière une roue et destiné à servir comme collecteur de particules projetées par la roue, des moyens d'aspiration permettant d'aspirer des phases gazeuses issues des particules à travers un tuyau muni d'un détecteur de gaz permettant d'identifier les gaz et ainsi la nature des particules. Ces moyens fonctionnent bien le temps que la concentration des gaz est suffisamment forte.

Dans ce contexte, la présente invention a pour but de pallier les défauts mentionnés ci-dessus.

A cette fin, le système de l'invention, par ailleurs conforme à la définition générique qu'en donne le préambule ci-dessus, se distingue particulièrement du fait que l'émetteur et le récepteur sont conformés pour être disposés à l'arrière du véhicule, le dispositif de détection comprenant en outre des moyens de déflection destinés à faire remonter, lorsque le véhicule se déplace, une veine d'air s'étendant entre le véhicule et le sol, vers le dispositif optique afin que la veine d'air traverse le faisceau optique.

Dans un mode de réalisation possible de l'invention, le chemin du faisceau comprend une partie transversale s'étendant immédiatement derrière le véhicule, les moyens de déflection comprenant un carénage s'étendant transversalement, disposé derrière ladite partie transversale du chemin du faisceau, et présentant un bord inférieur situé à proximité immédiate du sol.

De préférence, le carénage comprend une partie inférieure souple.

Avantageusement, la partie inférieure du carénage est en matière plastique.

Par exemple, le véhicule comprend un plancher délimitant la veine d'air, ladite partie transversale du faisceau s'étendant à un niveau supérieur au plancher du véhicule.

Avantageusement, le véhicule comprend un attelage pour remorque, l'émetteur, le récepteur et les moyens de déflection étant rigidement fixés de façon amovible sur cet attelage.

De préférence, les moyens de déflection comprennent un organe d'adaptation rigidement fixé à l'attelage, et une première armature de fixation rigide du carénage sur cet organe d'adaptation, l'émetteur et le récepteur étant rigidement fixés à cet organe d'adaptation par une seconde armature mécaniquement indépendante de la première.

Par exemple, le carénage et la première armature présentent une rigidité suffisante pour protéger l'émetteur et le récepteur en cas de choc à faible vitesse à l'arrière du véhicule.

Avantageusement, le système comprend un tube d'échappement adaptable de façon amovible sur le pot d'échappement du véhicule et débouchant à l'extérieur du carénage.

Avantageusement, le dispositif de détection comprend au moins un organe d'aspiration accélérant le débit de la veine d'air à travers le chemin du faisceau.

Selon un second aspect, l'invention concerne un dispositif optique de détection mis en oeuvre dans le système selon les caractéristiques ci-dessus.

D'autres caractéristiques et avantages de l'invention ressortiront clairement de la description qui en est faite ci-dessous, à titre indicatif et nullement limitatif, en référence aux figures annexées, parmi lesquelles :
- la figure 1 est une vue de côté du système selon l'invention,
- la figure 2 est une représentation schématique partielle du dispositif de détection du système de la figure 1,
- la figure 3 est une vue de dessus du dispositif de détection suivant la flèche III de la figure 1,
- la figure 4 est une vue de dessous et de côté du dispositif de détection suivant la flèche IV de la figure 2, une plaque latérale du carénage n'étant pas représentée,
- la figure 5 est une représentation graphique de la distribution d'écarts de réponse, exprimés en ppm en abscisse, entre un véhicule équipé d'un dispositif de détection optique et un véhicule de référence équipé d'un système de détection traditionnel avec aspiration et purification, la courbe en traits pointillés représentant la distribution d'écarts de réponse pour un véhicule dont le dispositif de détection optique est disposé à l'avant, la courbe en traits pleins représentant la distribution d'écarts de réponse pour le système de l'invention avec un dispositif de détection à l'arrière, et
- la figure 6 est une vue en coupe selon les flèches VI de la figure 3.

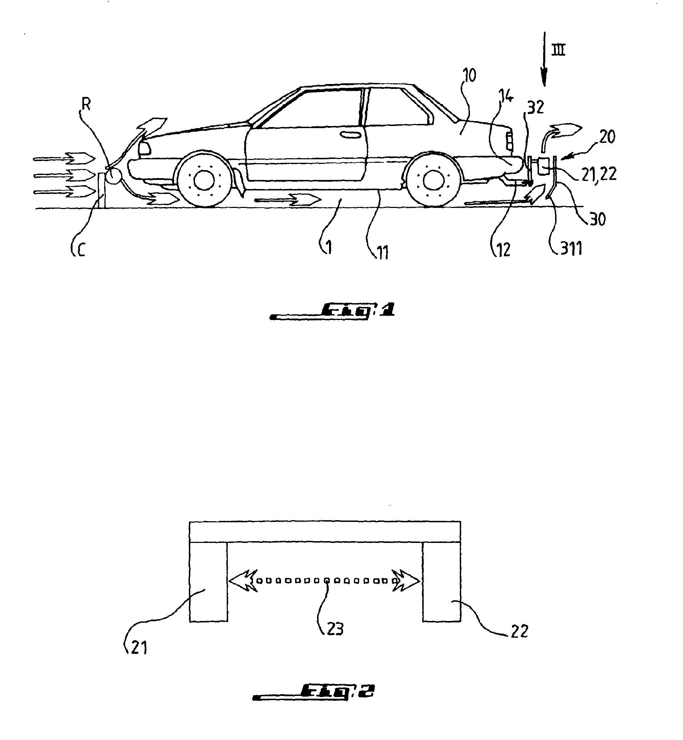

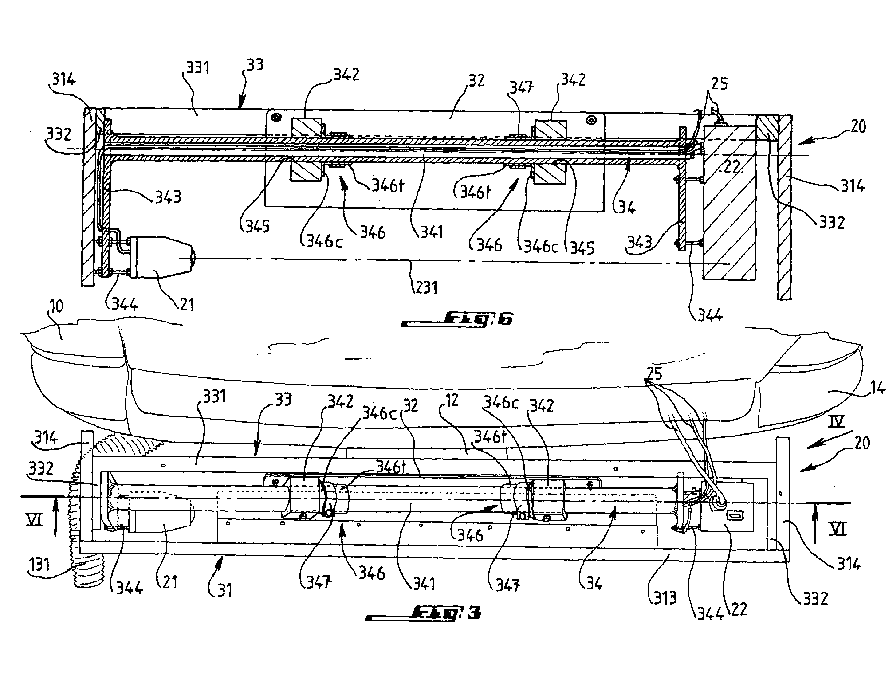

La figure 2 représente un système terrestre mobile de détection in situ d'un ou plusieurs gaz dans l'air, tel que du méthane, à proximité du sol, comprenant un véhicule 10 apte à se déplacer sur le sol, et un dispositif optique de détection du gaz 20 rigidement fixé sur le véhicule.

Le véhicule est typiquement une voiture automobile.

Le dispositif de détection 20 comprend, comme le montre la figure 2, un émetteur 21 fixe produisant un faisceau optique 23, un récepteur fixe 22 captant le faisceau optique 23 après que celui-ci ait parcouru un chemin prédéterminé, et des moyens pour détecter et quantifier le gaz sur ledit chemin en fonction des caractéristiques du faisceau 23 capté par le récepteur 22.

Le type de faisceau optique 23 utilisé, en particulier sa longueur d'onde, est fonction de la nature du gaz à détecter. Dans le cas du méthane, on utilise un faisceau infrarouge.

Une partie de ce faisceau 23 est absorbée par les molécules de gaz se trouvant sur le chemin du faisceau, ce qui permet de détecter la présence de gaz et de mesurer certaines caractéristiques, en particulier sa concentration.

L'émetteur 21 et le détecteur 22 sont installés en vis-à-vis, comme le montre la figure 1, le chemin du faisceau 23 étant alors une simple ligne droite.

D'autres montages sont possibles. Le détecteur 22 peut être disposé à côté de l'émetteur, un miroir étant disposé face à l'émetteur 21 et réfléchissant le faisceau vers le détecteur 22. Le chemin du faisceau 23 est alors constitué de deux lignes droites de sens inverses.

Selon l'invention, l'émetteur 21 et le récepteur 22 sont disposés à l'arrière du véhicule 10. Par ailleurs, le dispositif de détection 20 comprend des moyens de déflection 30 pour faire traverser le chemin du faisceau 23 par la plus grande partie d'une veine d'air 1 s'étendant entre le véhicule 10 et le sol quand le véhicule 10 se déplace.

Comme le montre la figure 1, le véhicule 10 comprend un pare-chocs arrière 14 sensiblement transversal, un plancher 11 délimitant la veine d'air 1, et un attelage pour remorque 12 disposé sous le pare-chocs arrière 14.

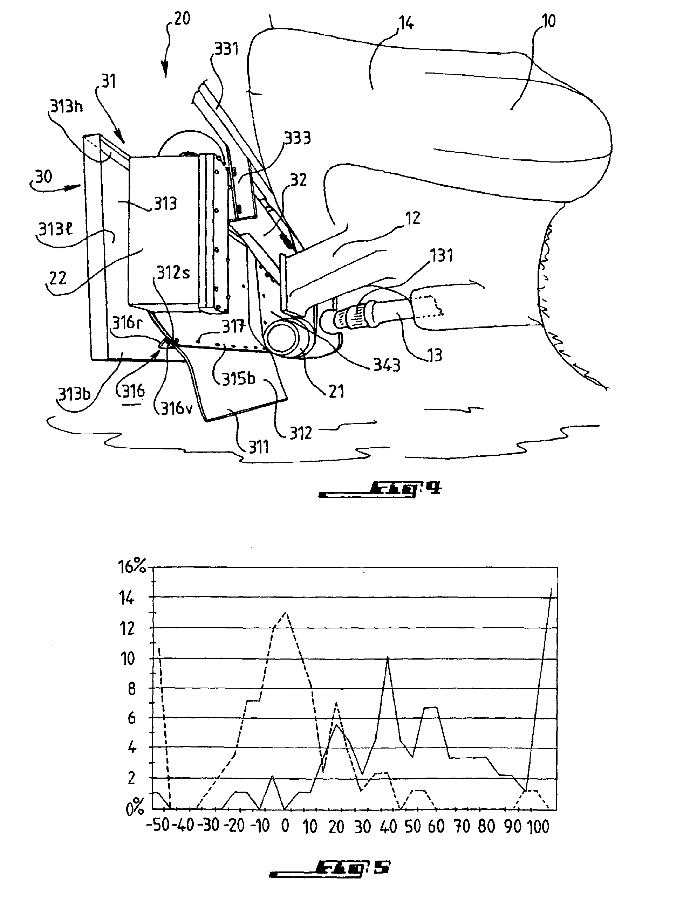

L'émetteur 21 et le détecteur 22 sont disposés immédiatement derrière ce pare-chocs arrière 14, en vis-à-vis l'un de l'autre suivant une direction transversale. Ils sont disposés des deux côtés latéraux opposés du véhicule, légèrement décalés vers le plan longitudinal médian du véhicule par rapport à ces côtés. Le chemin du faisceau 23 comprend donc une partie transversale 231 unique s'étendant immédiatement derrière le véhicule 10, comme on peut le voir sur les figures 3 et 6.

Cette partie transversale 231 est de préférence située à un niveau supérieur au plancher 11, de telle sorte que le détecteur et l'émetteur sont situés notamment au dessus du sol et ne courent pas le risque d'être endommagés par des obstacles se trouvant sur le chemin du véhicule.

Les moyens de déflection 30 sont représentés sur la figure 3 et comprennent un organe d'adaptation 32 rigidement fixé à l'attelage 12, un carénage 31 s'étendant transversalement, disposé derrière ladite partie transversale 231 du chemin du faisceau, et une première armature 33 de fixation rigide du carénage 31 sur l'organe d'adaptation 32.

Le carénage 31 comprend une plaque principale transversale 313, verticale, une partie inférieure souple 312 transversale disposée sous la plaque principale 313 et deux plaques latérales rigides 314 solidaires de la plaque principale 313.

La plaque principale 313, visible sur la figure 4, est rigide et présente une forme rectangulaire, délimitée par un bord haut 313h, un bord bas 313b parallèle et opposé au bord haut 313h et deux bords latéraux opposés 3131 mutuellement parallèles et perpendiculaires aux bords haut 313h et bas 313b. La plaque principale 313 est par exemple métallique.

Elle s'étend sur toute la largeur du véhicule 10 et présente une hauteur légèrement supérieure à celle du récepteur 22 et de l'émetteur 21. La plaque principale 313 est disposée sensiblement à la même hauteur que ceux-ci, de façon à les protéger en cas de choc arrière et forcer le passage de l'air dans le chemin du faisceau.

La partie inférieure 312 est une pièce rectangulaire, souple, en matière plastique. Elle est par exemple constituée de caoutchouc synthétique de forte épaisseur (plusieurs millimètres). Elle est fixée par un bord transversal supérieur 312s à la plaque transversale 313, et pend sous cette plaque. Elle présente un bord inférieur 311, parallèle et opposé au bord supérieur 312s, descendant à proximité immédiate du sol.

Le bord transversal supérieur 312s est pincé entre des plaques de fixation métalliques supérieure et inférieure 315 et 316 solidaires d'une face avant de la plaque principale 313 tournée vers le véhicule 10.

La plaque de fixation inférieure 316 s'étend transversalement et présente, en section dans un plan longitudinal vertical, la forme d'un chevron présentant un nez pointant vers le haut.

Cette plaque présente une aile arrière 316r solidaire de la plaque principale 313 par un bord transversal arrière inférieur. Ce bord est par exemple soudé sur la plaque principale 313. L'aile arrière 316r est inclinée vers le haut et l'avant à partir du bord arrière inférieur et présente un bord avant supérieur constituant un côté du nez du chevron.

La plaque de fixation inférieure 316 comprend également une aile avant 316v présentant un bord arrière supérieur solidaire du bord avant supérieur de l'aile arrière 316r. Ce bord arrière supérieur constitue un autre côté du nez. L'aile avant 316v est inclinée vers le bas et l'avant à partir du bord arrière supérieur et présente un bord avant inférieur libre.

La plaque de fixation inférieure 316 est fixée au bord bas 313b de la plaque principale 313.

La plaque de fixation supérieure 315 présente une forme générale rectangulaire et est plaquée sur la face avant de la plaque principale 313 par une moitié supérieure. Elle présente également une moitié inférieure prolongeant la moitié supérieure vers le bas en se décollant progressivement de la plaque principale 313. Cette moitié inférieure fait saillie vers l'avant par rapport à la plaque principale 313 et présente un bord avant transversal 316b venant se superposer exactement à l'aile avant 316v de la pièce de fixation inférieure 316.

Le bord transversal supérieur 312s est pris entre le bord avant transversal 315b et l'aile avant 315v. Des boulons 317 ou des rivets traversent le bord transversal supérieur 312s, le bord avant transversal 315b, et l'aile avant 315v et maintiennent le bord transversal supérieur 312s en pression entre le bord avant transversal 315b et l'aile avant 315v.

La partie inférieure 312 pend en dessous de la plaque principale 313, en étant légèrement inclinée vers l'avant et le bas à partir du bord bas 313b de cette plaque 313, comme le montre la figure 4.

Les plaques latérales 314 sont solidaires des bords latéraux opposés 3131 de la plaque principale 313 et s'étendent entre cette plaque et le véhicule 10. Elles s'étendent dans des plans verticaux longitudinaux mutuellement parallèles et sont de même hauteur que la plaque principale 313. Les plaques latérales 314 sont métalliques et sont soudées, rivetées ou vissées sur la plaque principale 313.

L'organe d'adaptation 32 est une plaque métallique transversale boulonnée sur l'attelage 12. La première armature 33 comprend une barre métallique transversale 331 solidaire de l'organe d'adaptation 32, et deux barres métalliques longitudinales 332 prolongeant la barre transversale 331 à des extrémités opposées. Les barres métalliques longitudinales 332 sont soudées à la barre transversale 331. La barre métallique transversale 331 est vissée sur l'organe d'adaptation 32 par l'intermédiaire de deux bras 333, soudés sur la barre 331 et s'étendant verticalement sous cette barre 331, comme le montre la figure 4.

Les barres longitudinales 332 s'étendent vers l'arrière à partir de la barre latérale 331, le long des plaques latérales 314. Elles sont fixées rigidement à ces plaques latérales 314, par exemple par vissage ou soudage.

L'émetteur 21 et le détecteur 22 sont rigidement fixés à l'organe d'adaptation 32 par une seconde armature 34, mécaniquement indépendante de la première, comprenant un tube creux transversal 341 portant l'émetteur 21 et le détecteur 22, et deux blocs supports 342 solidarisant le tube transversal 342 à l'organe d'adaptation 32. Cette seconde armature est représentée sur les figures 3 et 6.

L'émetteur 21 et le détecteur 22 sont rigidement fixés à des extrémités opposées du tube 341, de telle sorte que leurs parties actives respectives soient disposées sous le tube. Le chemin du faisceau s'étend donc parallèlement au tube, et sous celui-ci. Cette seconde armature est représentée sur les figures 3 et 6.

Les extrémités du tube 341 sont conformées en brides formant des plaques de fixation 343 pour l'émetteur 21 et le récepteur 22.

Ces plaques 343 s'étendent dans des plans verticaux longitudinaux.

Chacune présente une partie supérieure conformée en collerette entourant le tube 341 par le haut, et une partie inférieure s'étendant vers le bas, parallèlement aux plaques latérales 314. Cette partie inférieure descend pratiquement au même niveau que les plaques latérales 314.

Des tiges filetées 344 formant tirants fixent rigidement l'émetteur 21 à une des plaques de fixation 343. Ces tiges sont vissées par des extrémités libres dans des orifices conjugués ménagés dans l'émetteur 21 et traversent des trous formés dans la plaque de fixation 343. Un écrou et un contre-écrou vissés sur chaque tige filetée 344 sont disposés de part et d'autre de ces trous et enserrent la plaque de fixation 343.

Le récepteur 22 est fixé de la même façon sur la plaque 343 opposée à l'émetteur 21. De la même façon que précédemment, des tiges filetées 344 formant tirants sont vissées par des extrémités libres dans des orifices conjugués ménagés dans le récepteur 22. Ces tiges traversent des trous formés la plaque. Un écrou et un contre-écrou vissés sur chaque tige filetée 344 sont disposés de part et d'autre de ces trous et enserrent la plaque.

La seconde armature 34 comprend deux blocs supports 342 parallélépipédiques, fixés par des faces avant respectives sur l'organe d'adaptation 32 et percés de trous de passage 345 respectifs. Les blocs sont vissés ou soudés sur l'organe d'adaptation 32.

Les trous 345 sont cylindriques, d'axe transversal, et alignés.

Le tube de support 341 traverse ces trous 345, les blocs supports 342 étant disposés approximativement à un tiers et à deux tiers de la longueur du tube.

Deux manchons de fixation 346 sont enfilés sur le tube 341. Chaque manchon 346 comprend une partie tubulaire transversale 346t enfilée sur le tube, et une partie en disque 346c formant collerette autour d'une extrémité de la partie tubulaire 346t et venue de matière avec la partie tubulaire. Ces manchons sont réalisés en matière plastique rigide.

Ces manchons 346 sont disposés entre les deux blocs supports 342, en sens inverse l'un de l'autre, de telle sorte que les collerettes 346c viennent en appui sur des faces latérales des blocs supports 342 mutuellement en regard.

Des colliers de fixation 347 enserrent la partie tubulaire 346t autour du tube 341 et fixent les manchons de fixation 346 relativement à ce tube. Les collerettes 346t bloquent alors le tube 341 en translation suivant la direction transversale par rapport aux blocs supports 342, dans les deux sens opposés.

Le dispositif de détection 20 comprend des moyens de commande, non représentés, de l'émetteur 21 et du récepteur 22. Ces moyens de commande et les moyens pour détecter et quantifier le gaz recherché sont disposés à l'intérieur du véhicule. Des câbles de transmission 25 relient les moyens de commande et les moyens pour détecter et quantifier le gaz à l'émetteur et au récepteur.

Ces câbles sortent du véhicule sous l'ouvrant arrière, une partie d'entre eux courant à l'intérieur du tube 341 pour rejoindre l'émetteur 21.

On notera que le carénage 31 et la première armature 32 présentent une rigidité suffisante pour protéger l'émetteur 21 et le récepteur 22 en cas de choc à faible vitesse à l'arrière du véhicule 10.

Par ailleurs, le système comprend un tube d'échappement 131, représenté sur la figure 3, adaptable de façon amovible sur le pot d'échappement 13 du véhicule et débouchant à l'extérieur du carénage 31, relativement plus à l'arrière que la plaque principale 313. Les gaz d'échappement du véhicule ne peuvent donc pas passer entre l'émetteur 21 et le récepteur 22 et perturber la mesure.

Dans une variante de réalisation non représentée, les blocs 342 peuvent comprendre chacun deux demi-blocs assemblés l'un à l'autre par des vis, le tube 341 étant engagé entre les deux demi-blocs et comprimés entre ces deux demi-blocs, de telle sorte que le tube est bloqué en translation transversalement.

Enfin, le dispositif de détection 20 peut comprendre des organes d'aspiration, non représentés, accélérant le débit de la veine d'air à travers le chemin du faisceau.

Ces organes d'aspiration sont typiquement des ventilateurs disposés entre le carénage 31 et l'arrière du véhicule 10. Ces ventilateurs sont rigidement fixés sur la première armature 33.

On va maintenant détailler le fonctionnement du dispositif de détection.

Quand le véhicule se déplace, l'air incident relativement au véhicule se divise en deux flux, un flux passant au-dessus du véhicule et un flux passant en dessous du véhicule, la ligne de partage se situant approximativement au niveau du pare-chocs avant, comme le montre la figure 2.

Le flux passant sous le véhicule forme la veine d'air 1, délimitée par le plancher et le sol, qui débouche derrière le véhicule.

La veine d'air 1 rencontre la partie inférieure 312 qui bloque sa circulation vers l'arrière et est contrainte de remonter entre la plaque principale 313 et l'arrière du véhicule, ce qui la force à traverser le chemin du faisceau.

Le bord inférieur 311 de la partie inférieure 312 du carénage 31 frôle en permanence le sol. La fraction de la veine d'air passant sous la partie inférieure 312 est très faible.

Quand le véhicule est contraint de rouler à faible vitesse, le débit d'air entre le carénage et le véhicule peut être accéléré en mettant en service les ventilateurs 24.

La position inclinée vers l'avant de la partie inférieure 312 est favorable pour faire remonter l'air.

Les résultats obtenus à l'aide du système de l'invention sont très bons, comme on peut le constater sur la figure 5. Cette figure représente l'écart de résultat entre un système traditionnel et un système de détection à infrarouge. Le système traditionnel comprend des tubes de prélèvement de petites dimensions disposés sous le pare-chocs du véhicule. Ces tubes aspirent l'air, qui est ensuite purifié pour augmenter la proportion du gaz recherché, puis analysé.

Des essais ont été effectués avec un système selon l'invention et avec un véhicule équipé d'un ensemble émetteur/ récepteur monté à l'avant.

La courbe en traits pleins de la figure 5 correspond à l'écart de résultats entre le système de l'invention et le système traditionnel et celle en traits interrompus entre le véhicule avec émetteur/ récepteur à l'avant et le système traditionnel.

Les essais étaient effectués en simulant des fuites de canalisations de gaz naturel, en faisant varier la vitesse des véhicules portant les dispositifs de détection. A chaque essai, on calcule la différence entre la concentration de gaz, exprimée en parties par million (ppm), mesuré par le système de l'invention ou le véhicule avec émetteur/ récepteur à l'avant, et la concentration de gaz mesurée par le système traditionnel.

La figure 5 porte en abscisse les différences en ppm, et en ordonnée la proportion du nombre d'essais total, exprimée en pourcentage, qui a conduit à observer ces différences.

On observe que la courbe en traits interrompus est plutôt centrée sur 0. La réponse du système de détection infrarouge monté à l'avant du véhicule n'est pas meilleur que celle du système traditionnel. De plus, dans 11% des cas, l'écart est inférieur ou égal à moins 50 ppm, ce qui signifie que le système de détection infrarouge monté à l'avant du véhicule ne détecte pratiquement rien alors que le système traditionnel signale la présence de gaz.

Au contraire, on observe que la réponse du système de l'invention est quasiment toujours meilleure que celle du système traditionnel. Dans 15% des essais, l'écart est supérieur ou égal à 100 ppm, la réponse du système de l'invention étant importante alors que le système traditionnel ne détecte quasiment rien.

Cette meilleure sensibilité du dispositif de détection monté à l'arrière, comparé au dispositif de détection monté à l'avant, se comprend quand on considère la figure 1.

Le dispositif de détection monté à l'avant ne "voit" qu'un volume d'air limité, représenté par un rond R, situé immédiatement devant le pare-chocs avant. Au contraire, le dispositif de détection monté à l'arrière "voit" toute la colonne d'air qui passe sous la voiture représenté par le rectangle C, c'est-à-dire toute la colonne d'air située entre le pare-chocs avant et le sol.

On comprend donc bien que le système de l'invention est particulièrement avantageux puisqu'il offre une sensibilité supérieure à celle des systèmes auxquels il a été comparé.

Par ailleurs, le dispositif de détection, y compris le carnage 31, peut être monté et démonté du véhicule simplement et rapidement, son seul point d'ancrage étant l'attelage pour remorque. Cette possibilité est nécessaire pour que le positif de détection soit considéré comme un accessoire démontable placé à l'arrière du véhicule et à ce titre ne réclame pas une homologation vis-à-vis du code de la route par les services compétents.

Seule la partie inférieure 312 du carénage est susceptible d'entrer en contact avec le sol. Cette partie est constituée de matière plastique, et son remplacement est peu onéreux.

La partie sensible du dispositif de détection est protégée en cas de choc arrière par le carénage.

A cet effet, le carénage est une pièce métallique. Le carénage, et d'autres pièces des moyens de déflection, peuvent également être réalisés en matière plastique, sans réserve que la rigidité des moyens de déflection soit suffisante en cas de choc.

Enfin, l'armature de l'émetteur et du récepteur est indépendante de celle du carénage, ce qui fait que les chocs, vibrations et frottements subis par ce carénage sont transmis de façon atténuée à l'émetteur et au récepteur et ne perturbent pas la mesure.

## Revendications

1. Dispositif optique (20) de détection in situ d'un ou plusieurs gaz dans l'air, tel que du méthane, à proximité du sol, conformé pour être monté sur un véhicule (10) apte à se déplacer sur le sol, le dispositif (20) étant alors rigidement fixé sur le véhicule (10), et comprenant un émetteur fixe (21) destiné à envoyer un faisceau optique (23) vers un récepteur fixe (22) disposé par rapport à l'émetteur fixe (21) de manière à capter le faisceau optique (23) après que celui-ci ait parcouru un chemin prédéterminé, et des moyens pour détecter et quantifier le gaz sur ledit chemin en fonction des caractéristiques du faisceau (23) capté par le récepteur (22), **caractérisé en ce que** l'émetteur (21) et le récepteur (22) sont conformés pour être disposés à l'arrière du véhicule (10), le dispositif de détection (20) comprenant en outre des moyens de déflection (30) destinés à faire remonter, lorsque le véhicule (10) se déplace, une veine d'air (1) s'étendant entre le véhicule (10) et le sol, vers le dispositif optique (20) afin que la veine d'air (1) traverse le faisceau optique (23).

2. Dispositif selon la revendication 1, **caractérisé en ce que** le chemin du faisceau (23) comprend une partie transversale (231) s'étendant, lorsque le dispositif optique (20) est monté sur le véhicule (10), immédiatement derrière le véhicule (10), les moyens de déflection (30) comprenant un carénage (31) s'étendant transversalement, disposé derrière ladite partie transversale (231) du chemin du faisceau (23), et présentant un bord inférieur (311) situé à proximité immédiate du sol.

3. Dispositif selon la revendication 2, **caractérisé en ce que** le carénage (31) comprend une partie inférieure (312) souple.

4. Dispositif selon la revendication 3, **caractérisé en ce que** la partie inférieure (312) du carénage (31) est en matière plastique.

5. Dispositif selon l'une quelconque des revendications 2 à 4, **caractérisé en ce qu'**il est conformé pour être monté à l'arrière d'un véhicule (10) comprenant un plancher (11) délimitant la veine d'air (1), de manière que ladite partie transversale (231) du faisceau (23) s'étendant à un niveau supérieur au plancher (11) du véhicule (10).

6. Dispositif selon l'une quelconque des revendications 2 à 5, **caractérisé en ce qu'**il est conformé pour être monté à l'arrière d'un véhicule (10) comprenant un attelage pour remorque (12), l'émetteur (21), le récepteur (22) et les moyens de déflection (30) étant destinés à être rigidement fixés de façon amovible sur cet attelage.

7. Dispositif selon la revendication 6, **caractérisé en ce que** les moyens de déflection (30) comprennent un organe d'adaptation (32) destinée à être rigidement fixé à l'attelage (12), et une première armature (33) de fixation rigide du carénage (31) sur cet organe d'adaptation (32), l'émetteur (21) et le récepteur (22) étant rigidement fixés à cet organe d'adaptation (32) par une seconde armature (34) mécaniquement indépendante de la première .

8. Dispositif selon la revendication 7, **caractérisé en ce que** le carénage (31) et la première armature (33) sont rigides afin de protéger l'émetteur (21) et le récepteur (22) en cas de choc à l'arrière du véhicule (10).

9. Dispositif selon l'une quelconque des revendications 2 à 8, **caractérisé en ce qu'**il comprend un tube d'échappement (131) adaptable de façon amovible sur le pot d'échappement (13) du véhicule (10) et débouchant à l'extérieur du carénage (31).

10. Dispositif selon l'une quelconque des revendications 2 à 9, **caractérisé en ce qu'**il comprend au moins un organe d'aspiration (24) accélérant le débit de la veine d'air (1) à travers le chemin du faisceau (23).

11. Système terrestre mobile de détection in situ d'un ou plusieurs gaz dans l'air, tel que du méthane, à proximité du sol, comprenant un véhicule (10) apte à se déplacer sur le sol et un dispositif optique (20) selon l'une quelconque des revendications 1 à 10, ce dispositif étant rigidement fixé à l'arrière du véhicule (10).

## Claims

1. Optical device (20) for detecting in situ one or several gases in the air, such as methane, near the ground, made to be fitted to a vehicle (10) able to move over the ground, the device (20) being fixed rigidly to the vehicle (10), and comprising a fixed transmitter (21) intended to send a light beam (23) towards a fixed receiver (22) arranged in relation to the fixed transmitter (21) so as to capture the light beam (23) after it has covered a predetermined path and means to detect and quantify the gas on this path according to the characteristics of the beam (23) captured by the receiver (22), **characterised in that** the transmitter (21) and the receiver (22) are made to be arranged at the back of the vehicle (10), the detection device (20) also comprising means of deflection (30) intended to have a stream of air (1) extending between the vehicle (10) and the ground brought towards the optical device (20) when the vehicle (10) moves so that the stream of air (1) crosses the light beam (23).

2. Device according to claim 1, **characterised in that** the path of the beam (23) comprises a transverse part (231) extending immediately behind the vehicle (10) when the optical device (20) is fitted to the vehicle (10), means of deflection (30), comprising a fairing (31) extending transversely, arranged behind this transverse part (23) of the path of the beam (23), having a bottom edge (311) situated right next to the ground.

3. Device according to claim 2, **characterised in that** the fairing (31) comprises a flexible bottom part (312).

4. Device according to claim 3, **characterised in that** the bottom part (312) of the fairing (31) is in plastic material.

5. Device according to any one of claims 2 to 4, **characterised in that** it is made to be fitted to the back of a vehicle (10), comprising a floor (11) delimiting the stream of air (1) so that this transverse part (231) of the beam (23) extends at a level above the floor (11) of the vehicle (10).

6. Device according to any one of claims 2 to 5, **characterised in that** it is made to be fitted to the back of a vehicle (10), comprising an attachment for towing (12), the transmitter (21), the receiver (22) and the means of deflection (30) being intended to be fixed rigidly to this attachment in a moveable way.

7. Device according to claim 6, **characterised in that** the means of deflection (30) comprise an adaptation device (32) intended to be fixed rigidly to the attachment (12) and a first frame (33) for fixing the fairing (31) rigidly to this adaptation device (32), the transmitter (21) and the receiver (22) being fixed rigidly to this adaptation device (32) by a second frame (34) mechanically independent of the first.

8. Device according to claim 7, **characterised in that** the fairing (31) and the first frame (33) are rigid in order to protect the transmitter (21) and the receiver (22) in the case of an impact to the back of the vehicle (10).

9. Device according to any one of claims 2 to 8, **characterised in that** it comprises an exhaust pipe (131) which can be adapted to the exhaust silencer (13) of the vehicle (10) in a moveable way, discharging on the outside of the fairing (31).

10. Device according to any one of claims 2 to 9, **characterised in that** it comprises at least one suction device (24) accelerating the flow of the stream of air (1) through the path of the beam (23).

11. Terrestrial mobile system for detecting in situ one or several gases in the air, such as methane, near the ground, comprising a vehicle (10) able to move over the ground and an optical device (20) according to any one of claims 1 to 10, this device being fixed rigidly to the back of the vehicle (10).

## Patentansprüche

1. Optische Vorrichtung (20) zum Erfassen in situ von einem oder mehreren Gasen in der Luft, wie etwa Methan, in Bodennähe, dazu ausgebildet, an einem am Boden fahrbaren Fahrzeug (10) montiert zu werden, wobei die Vorrichtung (20) dabei starr an dem Fahrzeug (10) befestigt wird und einen feststehenden Sender (21) enthält, der dazu bestimmt ist, einen optischen Strahl (23) zu einem feststehenden Empfänger (22) zu senden, der so bezüglich des feststehenden Senders (21) angeordnet ist, dass er den optischen Strahl (23) empfängt, nachdem dieser einen vorbestimmten Weg zurückgelegt hat, sowie Mittel zum Erfassen und Quantifizieren von Gas über den Weg in Abhängigkeit von den Eigenschaften des über den Empfänger (22) empfangenen Strahls (23), **dadurch gekennzeichnet, dass** der Sender (21) und der Empfänger (22) dazu ausgebildet sind, am Heck des Fahrzeugs (10) angeordnet zu werden, wobei die Erfassungsvorrichtung (20) ferner Umlenkmittel (30) enthält, die dazu bestimmt sind, bei fahrendem Fahrzeug (10) einen sich zwischen Fahrzeug (10) und Boden erstreckenden Luftstrom (1) zur optischen Vorrichtung (20) hin anzuheben, damit der Luftstrom (1) den optischen Strahl (23) durchquert.

2. Vorrichtung nach Anspruch 1, **dadurch gekennzeichnet, dass** die Wegstrecke des Strahls (23) einen quer verlaufenden Abschnitt (231) enthält, der bei am Fahrzeug (10) montierter optischer Vorrichtung (20) unmittelbar hinter dem Fahrzeug (10) verläuft, wobei die Umlenkmittel (30) eine quer verlaufende Verkleidung (31) enthalten, die hinter dem quer verlaufenden Abschnitt (231) der Wegstrecke des Strahls (23) angeordnet ist und eine Unterkante (311) aufweist, die sich in unmittelbarer Nähe des Bodens befindet.

3. Vorrichtung nach Anspruch 2, **dadurch gekennzeichnet, dass** die Verkleidung (31) ein nachgiebiges Unterteil (312) enthält.

4. Vorrichtung nach Anspruch 3, **dadurch gekennzeichnet, dass** das Unterteil (312) der Verkleidung (31) aus Kunststoff besteht.

5. Vorrichtung nach einem der Ansprüche 2 bis 4, **dadurch gekennzeichnet, dass** sie dazu ausgebildet ist, am Heck eines Fahrzeugs (10) montiert zu werden, das einen Fahrzeugboden (11) enthält, der den Luftstrom (1) begrenzt, so dass der quer verlaufende Abschnitt (231) des Strahls (23) in einem höheren Bereich als der Fahrzeugboden (11) des Fahrzeugs (10) verläuft.

6. Vorrichtung nach einem der Ansprüche 2 bis 5, **dadurch gekennzeichnet, dass** sie dazu ausgebildet ist, am Heck eines Fahrzeugs (10) montiert zu werden, das eine Anhängerkupplung (12) enthält, wobei der Sender (21), der Empfänger (22) und die Umlenkmittel (30) dazu bestimmt sind, abnehmbar an dieser Kupplung starr befestigt zu werden.

7. Vorrichtung nach Anspruch 6, **dadurch gekennzeichnet, dass** die Umlenkmittel (30) ein Adapterglied (32) enthalten, das dazu bestimmt ist, starr an der Kupplung (12) befestigt zu werden, sowie eine erste Armierung (33) zum starren Befestigen der Verkleidung (31) an diesem Adapterglied (32), wobei der Sender (21) und der Empfänger (22) über eine zweite Armierung (34) starr an diesem Adapterglied (32) befestigt sind, welche mechanisch unabhängig von der ersten ist.

8. Vorrichtung nach Anspruch 7, **dadurch gekennzeichnet, dass** die Verkleidung (31) und die erste Armierung (33) starr sind, um den Sender (21) und den Empfänger (22) bei einem Stoß auf das Heck des Fahrzeugs (10) zu schützen.

9. Vorrichtung nach einem der Ansprüche 2 bis 8, **dadurch gekennzeichnet, dass** sie ein Auspuffrohr (131) enthält, das abnehmbar an dem Auspufftopf (13) des Fahrzeugs (10) anpassbar ist und nach außerhalb der Verkleidung (31) mündet.

10. Vorrichtung nach einem der Ansprüche 2 bis 9, **dadurch gekennzeichnet, dass** sie zumindest ein Saugglied (24) enthält, welches den Luftstromdurchsatz (1) durch die Wegstrecke des Strahls (23) beschleunigt.

11. Erdgebundenes, bewegliches System zum Erfassen in situ von einem oder mehreren Gasen in der Luft, wie etwa Methan, in Bodennähe, mit einem am Boden fahrbaren Fahrzeug (10) und einer optischen Vorrichtung (20) nach einem der Ansprüche 1 bis 10, wobei diese Vorrichtung starr am Heck des Fahrzeugs (10) befestigt ist.
